# EUROPEAN PATENT SPECIFICATION

(11) **EP 3 648 810 B1**
(45) Date of publication and mention of the grant of the patent: **17.09.2025**
(21) Application number: 18740523.8
(22) Date of filing: 02.07.2018
(51) Int. Cl.: A61M 1/00

(54) **WOUND THERAPY SYSTEM AND DRESSING FOR DELIVERING OXYGEN TO A WOUND**
WUNDTHERAPIESYSTEM UND VERBAND ZUR ABGABE VON SAUERSTOFF AN EINE WUNDE
SYSTÈME DE TRAITEMENT DE PLAIE ET PANSEMENT POUR ADMINISTRER DE L'OXYGÈNE À UNE PLAIE

(30) Priority: 07.07.2017 US 201762529489 P; 25.08.2017 US 201762550236 P
(43) Date of publication of application: 13.05.2020
(73) Proprietor: Smith & Nephew plc, Watford, Hertfordshire WD18 8YE (GB)
(72) Inventor: HARTWELL, Edward, Yerbury, Hull HU3 2BN (GB); WEBSTER, Iain, Hull HU3 2BN (GB)
(74) Representative: Guy, Mark Robert
(86) International application number: PCT/EP2018/067752
(87) International publication number: WO 2019/007874

(56) References cited:
- US-A1- 2014 107 562
- US-A1- 2015 094 646
- US-A1- 2017 143 881

## Description

### BACKGROUND

### Technical Field

Embodiments described herein relate to apparatuses, systems, and methods the treatment of wounds, for example using dressings in combination with oxygen.

### Description of the Related Art

The treatment of open or chronic wounds that are too large to spontaneously close or otherwise fail to heal has proven to be persistently troublesome. Oxygen can play an important role in wound treatment and wound healing due to the increased demand for reparative processes such as cell proliferation, bacterial defense, angiogenesis, and collagen synthesis. Nearly every phase of wound healing can benefit from presence of oxygen.

Oxygen can be essential for the production of biological energy equivalents, such as, adenosine triphosphate (ATP), in aerobic glycolysis, the citric acid cycle, and the oxidation of fatty acids. Therefore, sufficient oxygenation of tissue is considered to be a prerequisite for adequate energy levels, which can be essential for proper cellular function. In healing tissue, sufficient oxygenation can be particularly relevant because of the increased energy demand for reparative processes such as cell proliferation, bacterial defense and collagen synthesis. The strictly oxygen-dependent nicotinamide adenine dinucleotide phosphate (NADPH)-linked oxygenase represents a further highly important enzyme in wound healing as it catalyzes the production of reactive oxygen species (ROS), such as peroxide anion (HO₂⁻), hydroxyl ion (HO⁻), and superoxide anion (O₂⁻). ROS play a prominent role in oxidative bacterial killing and co-regulates prevalent processes in wound healing such as cytokine release, cell proliferation and angiogenesis.

On the other hand, oxygen deficiency or hypoxia can degrade wound healing. The initial implication of hypoxia on the molecular level can cause the impairment of mitochondrial oxidative phosphorylation with a subsequently reduced ATP production. As a consequence, ATP-dependent membrane transport proteins such as Na⁺/K⁺-ATPase or Ca⁺⁺-ATPase can drop out, which can lead to a loss of the transmembrane potential with subsequent cell swelling. Intracellular accumulation of calcium ions can activate a signal transduction pathway that ends up in cell membrane disruption, which can result in a promotion of inflammatory cascades via various signal pathways. Proinflammatory cytokines and chemokines such as tumour necrosis factor (TNF) and IL-1 can be released, which can attract and activate neutrophils and macrophages. In addition, hypoxia can induce a pronounced expression of endothelial adhesion molecules such as intercellular adhesion molecule-1, vascular cell adhesion molecule-1, and the corresponding ligands leucocyte function-associated antigen-1 and very late antigen-4 that enhance the extravasation and invasion of neutrophils and macrophages into the wound site with a subsequent autocrine synthesis of proinflammatory cytokines such as IL-1α, IL-1β, IL-6 and TNF. Growth factors and cytokines can be released in a self-perpetuating manner, macrophages can be continuously attracted, and tissue degenerating enzymes can be continuously generated.

However, many existing wound healing systems and wound dressings are inadequate to ensure that oxygen can be dependably delivered to the wound. Accordingly, there is need for improved systems, dressing, and methods for supplying oxygen to a wound to facilitate wound healing.

US2014/107562 A1 discloses oxygen diffusive wound dressings and methods of manufacturing and use. The wound dressing may generally provide a ready supply of oxygen to a wound being treated via one or more oxygen conduits which are designed to pass oxygen from ambient air or other oxygen reservoirs into proximity to the wound, and may also provide for exudate removal through transecting channels in fluid communication with both the wound surface and a hydrophilic absorbent material. US 2017/143881 A1 discloses wound healing systems, which deliver oxygen to the wound bed and apply negative pressure thereto, where the source of oxygen and negative pressure are simultaneously applied to distal sites of the dressing. US 2015/094646 A1 discloses an oxygen distributor positionable, in use, in a wound for supplying oxygen to the wound has an oxygen delivery area for, in use, receiving a supply of oxygen. At least one tube extends from the oxygen delivery area, having a tube wall with an oxygen-permeable, liquid-impermeable section. Oxygen delivered to the oxygen delivery area can flow away from the oxygen delivery area along the, or each, tube.

### SUMMARY

In embodiments according to the invention, a wound treatment apparatus is provided according to claim 1.

The apparatus of the preceding paragraph may also include any combination of the features described in the following paragraphs, among others described herein. Each of the features described in the following paragraphs may also be part of another embodiment that does not necessarily include all of the features of the previous paragraph.

The transmission layer can include at least one of a material with three dimensional structure or an acquisition distribution layer. The absorbent layer can include an orifice configured to provide direct fluidic communication between the oxygen source and the transmission layer. The wound contact layer can include adhesive on a wound facing side, the adhesive configured to provide a substantially gas tight seal over the wound. The adhesive can be silicone adhesive. The wound contact layer can include a plurality of slits configured to distribute oxygen over the wound and further configured to transmit wound fluid away from the wound. The backing layer can be substantially oxygen and fluid impermeable. The backing layer can include ethylene vinyl alcohol (EVA).

The backing layer can include another orifice configured to facilitate supply of negative pressure to the wound. A negative pressure source configured to supply negative pressure to the wound through the another orifice can be included. The wound dressing can further include a filter configured to prevent wound fluid from reaching the oxygen source. A pressure sensor can be positioned in a fluid flow path comprising the oxygen source and the wound dressing and a controller configured to regulate supply of oxygen based on feedback from the pressure sensor can be included. The controller can be further configured to regulate application of negative pressure to the wound. The controller can be further configured to regulate application of simultaneous supply of oxygen and negative pressure to the wound. The controller can be further configured to regulate application of sequential supply of oxygen and negative pressure to the wound.

Any of the features, components, or details of any of the arrangements or embodiments disclosed in this application, including without limitation any of the wound therapy apparatuses or dressing described below, are interchangeably combinable with any other features, components, or details of any of the arrangements or embodiments disclosed herein to form new arrangements and embodiments.

### BRIEF DESCRIPTION OF THE DRAWINGS

Embodiments of the present disclosure will now be described hereinafter, by way of example only, with reference to the accompanying drawings in which:
Figure 1 illustrates an embodiment of a wound treatment system for delivering oxygen to a wound employing a flexible fluidic connector and a wound dressing capable of absorbing and storing wound exudate;
Figure 2A illustrates an embodiment of a wound treatment system for delivering oxygen to a wound employing a flexible fluidic connector and a wound dressing capable of absorbing and storing wound exudate;
Figure 2B illustrates a cross section of an embodiment of a fluidic connector connected to a wound dressing; and
Figures 3A-D illustrate the use and application of an embodiment of a wound treatment system onto a patient.

### DETAILED DESCRIPTION

### Overview

Embodiments disclosed herein relate to apparatuses and methods of treating a wound with oxygen, including a source of oxygen, which may supply positive pressure, or negative pressure and wound dressing components and apparatuses. The apparatuses and components comprising the wound overlay and packing materials, if any, are sometimes collectively referred to herein as dressings.

Certain embodiments disclosed herein relate to wound therapy for a human or animal body. Therefore, any reference to a wound herein can refer to a wound on a human or animal body, and any reference to a body herein can refer to a human or animal body. The term "wound" as used herein, in addition to having its broad ordinary meaning, includes any body part of a patient that may be treated using, for example, oxygen or negative pressure. It is to be understood that the term wound is to be broadly construed and encompasses open and closed wounds in which skin is torn, cut or punctured or where trauma causes a contusion, or any other superficial or other conditions or imperfections on the skin of a patient or otherwise that benefit from oxygen or reduced pressure treatment. A wound is thus broadly defined as any damaged region of tissue where fluid may or may not be produced. Examples of such wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, stoma, surgical wounds, trauma and venous ulcers or the like.

Treatment of such wounds can be performed by supplying oxygen to a wound to facilitate and promote healing of the wound. In some embodiments, negative pressure wound therapy can be applied simultaneously or sequentially to further facilitate and promote healing of the wound. It will also be appreciated that the systems, wound dressings, and methods as disclosed herein may be applied to other parts of the body, and are not necessarily limited to treatment of wounds.

It will be understood that embodiments of the present disclosure can be generally applicable in wound healing systems that may, in addition to oxygen therapy, also provide topical negative pressure ("TNP") therapy. Briefly, oxygen or negative pressure wound therapy assists in the closure and healing of many forms of "hard to heal" wounds by reducing tissue oedema; encouraging blood flow and granular tissue formation; reducing bacterial load (and thus infection risk); and removing excess exudate. In addition, the therapy allows for less disturbance of a wound leading to more rapid healing. Wound healing systems may also assist in the healing of surgically closed wounds, grafts, and flaps, among others.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g., -40 mmHg is less than -60 mmHg). Negative pressure that is "more" or "greater" than -X mmHg corresponds to pressure that is further from atmospheric pressure (e.g., -80 mmHg is more than -60 mmHg). In some embodiments, local ambient atmospheric pressure is used as a reference point, and such local atmospheric pressure may not necessarily be, for example, 760 mmHg.

Further, positive pressure levels, such as X mmHg, represent pressure levels relative to normal ambient atmospheric pressure. Accordingly, a positive pressure value of X mmHg reflects absolute pressure that is X mmHg above 760 mmHg or, in other words, an absolute pressure of (760+X) mmHg. In addition, positive pressure that is "less" or "smaller" than X mmHg corresponds to pressure that is closer to atmospheric pressure (e.g., 40 mmHg is less than 60 mmHg). Positive pressure that is "more" or "greater" than X mmHg corresponds to pressure that is further from atmospheric pressure (e.g., 80 mmHg is more than 60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, -200 mmHg would be about 560 mmHg in practical terms. In some embodiments, the pressure range can be between about -40 mmHg and -150 mmHg. Alternatively a pressure range of up to -75 mmHg, up to -80 mmHg or over -80 mmHg can be used. Also in other embodiments a pressure range of below -75 mmHg can be used. Alternatively, a pressure range of over approximately -100 mmHg, or even -150 mmHg, can be supplied by the negative pressure apparatus.

The positive pressure range for some embodiments of the present disclosure can be approximately 20 mmHg, or between approximately 10 mmHg and 40 mmHg. As these pressures are relative to normal ambient atmospheric pressure, for example, 20 mmHg would be 780 mmHg. In some implementations, positive pressure of less than 10 mmHg or more than 40 mmHg can be used.

In some embodiments of wound closure devices described herein, increased wound contraction can lead to increased tissue expansion in the surrounding wound tissue. This effect may be increased by varying the force applied to the tissue, for example by varying the positive (for example, due to oxygen delivery) or negative pressure applied to the wound over time, possibly in conjunction with increased tensile forces applied to the wound via embodiments of the wound closure devices. In some embodiments, positive or negative pressure may be varied over time for example using a sinusoidal wave, square wave, or in synchronization with one or more patient physiological indices (e.g., heartbeat). Examples of such applications where additional disclosure relating to the preceding may be found in U.S. Patent No. 8,235,955, titled "Wound treatment apparatus and method," issued on August 7, 2012; and U.S. Patent No. 7,753,894, titled "Wound cleansing apparatus with stress," issued July 13, 2010.

Embodiments of the wound dressings, wound dressing components, wound treatment apparatuses and methods described herein may also be used in combination or in addition to those described in International Application No. PCT/IB2013/001469, filed May 22, 2013, published as WO 2013/175306 A2 on November 28, 2013, titled "APPARATUSES AND METHODS FOR NEGATIVE PRESSURE WOUND THERAPY," U.S. Patent Application No. 14/418874, filed January 30, 2015, published as US 2015/0190286 A1 on July 9, 2015, titled "WOUND DRESSING AND METHOD OF TREATMENT,". Embodiments of the wound dressings, wound treatment apparatuses and methods described herein may also be used in combination or in addition to those described in U.S. Patent Application No. 13/092,042, filed April 21 2011, published as US2011/0282309, titled "WOUND DRESSING AND METHOD OF USE," and U.S. Patent Application No. 14/715,527, filed May 18, 2015, published as US2016/0339158, titled "FLUIDIC CONNECTOR FOR NEGATIVE PRESSURE WOUND THERAPY", including further details relating to embodiments of wound dressings, the wound dressing components and principles, and the materials used for the wound dressings.

Additionally, some embodiments related to TNP wound treatment comprising a wound dressing in combination with a pump or associated electronics described herein may also be used in combination or in addition to those described in International Application No. PCT/EP2016/059329, filed April 26, 2016, published as WO2016174048 A1 on November 3, 2016, titled "REDUCED PRESSURE APPARATUS AND METHODS."

### Wound Therapy Systems and Dressings

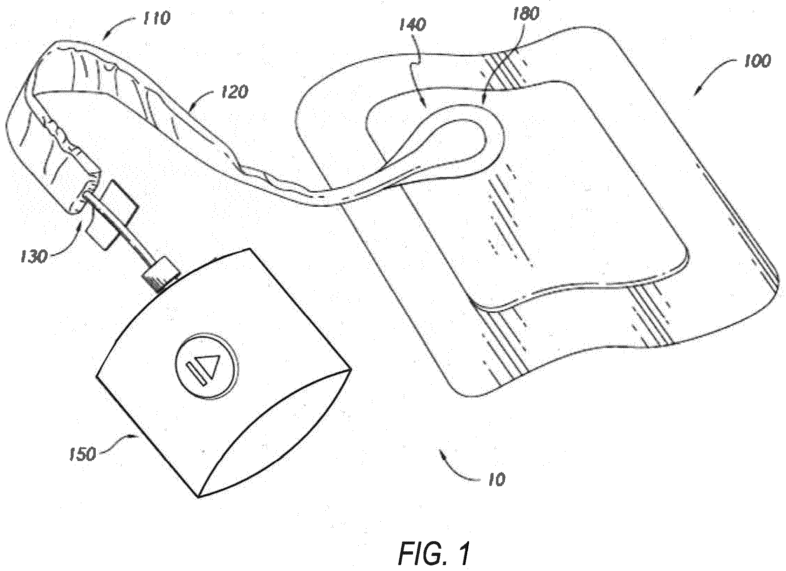

Figure 1 illustrates an embodiment of a wound therapy or treatment system 10 employing a wound dressing 100 in conjunction with a fluidic connector 110. Here, the fluidic connector 110 may comprise an elongate conduit, more preferably a bridge 120 having a proximal end 130 and a distal end 140, and an applicator 180 at the distal end 140 of the bridge 120. An optional coupling is preferably disposed at the proximal end 130 of the bridge 120. A cap may be provided with the system to prevent fluids from leaking out of the proximal end 130. The system 10 may include an oxygen reservoir or source 150 capable of supplying oxygen. The source 150 can be one or more of an oxygen concentrator, oxygen tank, or the like. For example, the source 150 can be an electrolytic (or electrochemical) oxygen generator, which may be fuel-cell powered (for example, utilizing nafion). The source 150 can include user interface or controls, such as a play/pause button as is illustrated in Figure 1, to control therapy. In some implementations, the source 150 can communicate with a remote computing device over a wired or wireless interface, receive commands from the remote device (such as, start/pause therapy), communicate data to the remote device (such as, therapy data), or the like. Communicated data, such as therapy data, can be used to monitor compliance with therapy, including oxygen therapy, negative pressure wound therapy, or the like.

In some embodiments, oxygen can be delivered at a positive pressure of about 20 mmHg. Delivery of oxygen to the wound may cause the tissue to oxygenate from about 20% to about 80% to 90% or more.

The source 150 may be connected to the coupling via a tube, or the source may be connected directly to the coupling or directly to the bridge 120. In use, the dressing 100 is placed over a suitably-prepared wound, which may in some cases be filled with a wound packing material such as foam or gauze. The applicator 180 of the fluidic connector 110 has a sealing surface that is placed over an aperture or port in the dressing 100 and is sealed to the top surface of the dressing 100. Either before, during, or after connection of the fluidic connector 110 to the dressing 100, the source 150 is connected, for example, via the tube to the coupling, or, as another example, is connected directly to the coupling or to the bridge 120. The source 150 is then activated, thereby supplying oxygen to the wound. Application of oxygen may be performed continuously or intermittently until a desired level of healing of the wound is achieved. In some embodiments, the source 150 can be miniaturized and portable. In some embodiments, the source 150 may be attached or mounted onto or adjacent the dressing 100.

In some embodiments, the source of oxygen and some or all other components of the system, such as power source(s), sensor(s), connector(s), user interface component(s) (such as button(s), switch(es), speaker(s), screen(s), etc.) and the like, can be integral with the wound dressing. The wound dressing can include a cover layer for positioning over the layers of the wound dressing. The cover layer can be the upper most layer of the dressing. In some embodiments, the wound dressing can include a second cover layer for positioning over the layers of the wound dressing and any of the integrated components. The second cover layer can be the upper most layer of the dressing or can be a separate envelope that encloses the integrated components of the wound therapy system.

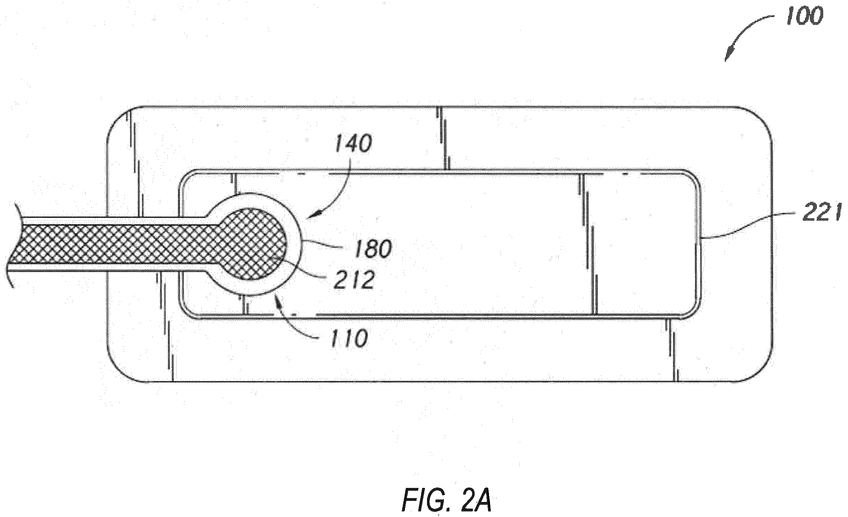

As shown in Figure 2A, the fluidic connector 110 comprises an enlarged distal end, or head 140 that is in fluidic communication with the dressing 100 as will be described in further detail below. In one embodiment, the enlarged distal end has a round or circular shape. The head 140 is illustrated here as being positioned near an edge of the dressing 100, but may also be positioned at any location on the dressing. For example, some embodiments may provide for a centrally or off-centered location not on or near an edge or corner of the dressing 100. In some embodiments, the dressing 100 may comprise two or more fluidic connectors 110, each comprising one or more heads 140, in fluidic communication therewith. In some embodiments, the head 140 may measure 30mm along its widest edge. The head 140 forms at least in part the applicator 180, described above, that is configured to seal against a top surface of the wound dressing.

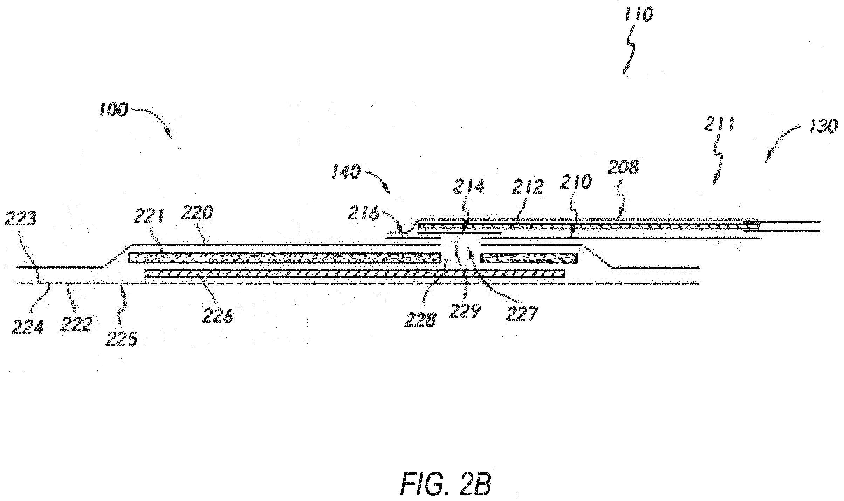

Figure 2B illustrates a cross-section through a wound dressing 100 similar to the wound dressing 100 as shown in Figure 1 and described in International Patent Publication WO2013175306 A2, filed May 22, 2013, entitled "APPARATUSES AND METHODS FOR NEGATIVE PRESSURE WOUND THERAPY", along with fluidic connector 110. The wound
dressing 100, which can alternatively be any wound dressing embodiment disclosed herein or any combination of features of any number of wound dressing embodiments disclosed herein, can be located over a wound site to be treated. The dressing 100 may be placed as to form a sealed cavity over the wound site. In some embodiments, the dressing 100 comprises a top or cover layer, or backing layer 220 attached to an optional wound contact layer 222, both of which are described in greater detail below. These two layers 220, 222 are preferably joined or sealed together so as to define an interior space or chamber. This interior space or chamber may comprise additional structures that may be adapted to distribute or transmit oxygen or negative pressure, store wound exudate and other fluids removed from the wound, and other functions which will be explained in greater detail below. Examples of such structures, described below, include a transmission layer 226 and an absorbent layer 221.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in Figure 2B, the wound contact layer 222 can be a polyurethane layer or polyethylene layer or other flexible layer which is perforated, for example via a hot pin process, laser ablation process, ultrasound process or in some other way or otherwise made permeable to liquid and gas. The wound contact layer 222 has a lower surface 224 and an upper surface 223. The perforations 225 preferably comprise through holes in the wound contact layer 222 which enable fluid to flow through the layer 222. The wound contact layer 222 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In certain embodiments, porosity of the wound contact layer 222 can vary from about 10% open area to about 95% open area. Different porosity of the wound contact layer 222 can correspond to different density or size of the perforations 225. In some configurations, the wound contact layer 222 may help maintain the integrity of the entire dressing 100 while also creating an air tight seal around the absorbent pad in order to supply oxygen to the wound or maintain negative pressure at the wound.

Some embodiments of the wound contact layer 222 may also act as a carrier for an optional lower and upper adhesive layer (not shown). For example, a lower pressure sensitive adhesive may be provided on the lower surface 224 of the wound dressing 100 whilst an upper pressure sensitive adhesive layer may be provided on the upper surface 223 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 100 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A layer 226 of porous material can be located above the wound contact layer 222. This porous layer, or transmission layer, 226 allows transmission of fluid including liquid or gas away from a wound site into upper layers of the wound dressing. In particular, the transmission layer 226 preferably ensures that an open air channel can be maintained to communicate oxygen or negative pressure over the wound area even when the absorbent layer has absorbed substantial amounts of exudates. The layer 226 should preferably remain open under the typical pressures that will be applied during oxygen therapy or negative pressure wound therapy as described above, so that the whole wound site sees an equalized positive or negative pressure. The layer 226 may be formed of a material having a three dimensional structure. For example, a knitted or woven spacer fabric (for example Baltex 7970 weft knitted polyester) or a non-woven fabric could be used.

In some embodiments, the transmission layer 226 comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 10 denier flat polyester and a third layer formed sandwiched between these two layers which is a region defined by a knitted polyester viscose, cellulose or the like monofilament fiber. Other materials and other linear mass densities of fiber could of course be used.

Whilst reference is made throughout this disclosure to a monofilament fiber it will be appreciated that a multistrand alternative could of course be utilized. The top spacer fabric thus has more filaments in a yarn used to form it than the number of filaments making up the yarn used to form the bottom spacer fabric layer.

This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent layer 221 helps lock the liquid away or itself wicks the liquid onwards towards the cover layer where it can be transpired.

Preferably, to improve the liquid flow across the transmission layer 226 (that is to say perpendicular to the channel region formed between the top and bottom spacer layers, the 3D fabric may be treated with a dry cleaning agent (such as, but not limited to, Perchloro Ethylene) to help remove any manufacturing products such as mineral oils, fats or waxes used previously which might interfere with the hydrophilic capabilities of the transmission layer. In some embodiments, an additional manufacturing step can subsequently be carried in which the 3D spacer fabric is washed in a hydrophilic agent (such as, but not limited to, Feran Ice 30g/l available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

In some embodiments, a wicking or acquisition distribution layer (ADL) can be included in addition to or instead of the transmission layer 226. ADL can horizontally wick fluid such as wound exudate as it is absorbed upward through the layers of the dressing 100. ADL can be positioned above the transmission layer 226, such as between the transmission layer and an absorbent layer 221, or in place of the transmission layer 226. Lateral wicking of fluid may allow maximum distribution of the fluid through the absorbent layer 221 and may enable the absorbent layer 221 to reach its full holding capacity. This may advantageously increase moisture vapor permeation and efficient delivery of oxygen or negative pressure to the wound site. Some embodiments of the ADL may comprise viscose, polyester, polypropylene, cellulose, or a combination of some or all of these, and the material may be needle-punched. Some embodiments of the ADL may comprise polyethylene in the range of 40-150 grams per square meter (gsm). In some embodiments, the ADL may have a thickness of 1.2 mm or about 1.2 mm, or may have a thickness in the range of 0.5 mm to 3.0 mm, or about 0.5 mm to about 3.0 mm.

A layer 221 of absorbent material is provided above the transmission layer 226. The absorbent material, which comprise a foam or non-woven natural or synthetic material, and which may optionally comprise a super-absorbent material, forms a reservoir for fluid, particularly liquid, removed from the wound site. In some embodiments, the absorbent layer 221 may also aid in drawing fluids towards the backing layer 220.

The material of the absorbent layer 221 may also prevent liquid collected in the wound dressing 100 from flowing freely within the dressing, and preferably acts so as to contain any liquid collected within the dressing. The absorbent layer 221 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when oxygen or negative pressure is supplied. Since in use the absorbent layer experiences positive or negative pressures, the material of the absorbent layer is chosen to absorb liquid under such circumstances. A number of materials exist that are able to absorb liquid when under positive or negative pressure, for example superabsorber material. The absorbent layer 221 may typically be manufactured from ALLEVYN^{™} foam, Freudenberg 114-224-4 or Chem-Posite^{™}11C-450. In some embodiments, the absorbent layer 221 may comprise a composite comprising superabsorbent powder, fibrous material such as cellulose, and bonding fibers. In a preferred embodiment, the composite is an airlaid, thermally-bonded composite.

In some embodiments, the absorbent layer 221 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 227 is provided in the backing layer 220 to allow oxygen or negative pressure to be supplied under the dressing 100 to the wound. The fluidic connector 110 is preferably attached or sealed to the top of the backing layer 220 over the orifice 227 made into the dressing 100, and communicates oxygen or negative pressure through the orifice 227. A length of tubing may be coupled at a first end to the fluidic connector 110 and at a second end to an oxygen source (not shown) to allow oxygen to be delivered to the wound. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 110 may be adhered and sealed to the backing layer 220 using an adhesive such as an acrylic, cyanoacrylate, epoxy, UV curable or hot melt adhesive. The fluidic connector 110 may be formed from a soft polymer, for example a polyethylene, a polyvinyl chloride, a silicone or polyurethane having a hardness of 30 to 90 on the Shore A scale. In some embodiments, the fluidic connector 110 may be made from a soft or conformable material.

Preferably the absorbent layer 221 includes at least one through hole 228 located so as to underlie the fluidic connector 110. The through hole 228 may in some embodiments be the same size as the opening 227 in the backing layer, or may be bigger or smaller. As illustrated in Figure 2B a single through hole can be used to produce an opening underlying the fluidic connector 110. It will be appreciated that multiple openings could alternatively be utilized. Additionally, should more than one port be utilized according to certain embodiments of the present disclosure one or multiple openings may be made in the absorbent layer and the obscuring layer in registration with each respective fluidic connector, which may be connected to one or more oxygen sources or negative pressure sources. Although not essential to certain embodiments of the present disclosure the use of through holes in the super-absorbent layer may provide a fluid flow pathway which remains unblocked in particular when the absorbent layer is near saturation.

The aperture or through-hole 228 is preferably provided in the absorbent layer 221 beneath the orifice 227 such that the orifice is connected directly to the transmission layer 226 as illustrated in Figure 2B. This allows oxygen or negative pressure applied to the fluidic connector 110 to be communicated to the transmission layer 226 without passing through the absorbent layer 221. This ensures that oxygen or negative pressure supplied to the wound site is not inhibited by the absorbent layer as it absorbs wound exudates. In other embodiments, no aperture may be provided in the absorbent layer 221, or alternatively a plurality of apertures underlying the orifice 227 may be provided. In further alternative embodiments, additional layers such as another transmission layer or an obscuring layer such as described in US Patent Publication 2015/0190286 A1, may be provided over the absorbent layer 221 and beneath the backing layer 220.

The backing layer 220 is preferably gas impermeable, but moisture vapor permeable, and can extend across the width of the wound dressing 100. The backing layer 220, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way, an effective chamber is made between the backing layer 220 and a wound site where oxygen can be supplied or a negative pressure can be established. In some implementations, the backing layer can include one or more polymers, such as ethylene vinyl alcohol (EVA), that are less permeable to oxygen, while being substantially moisture vapor permeable.

The backing layer 220 is preferably sealed to the wound contact layer 222 in a border region around the circumference of the dressing, ensuring that no air is drawn in through the border area, for example via adhesive or welding techniques. The backing layer 220 protects the wound from external bacterial contamination (bacterial barrier) and allows liquid from wound exudates to be transferred through the layer and evaporated from the film outer surface. The backing layer 220 preferably comprises two layers; a polyurethane film and an adhesive pattern spread onto the film. The polyurethane film is preferably moisture vapor permeable and may be manufactured from a material that has an increased water transmission rate when wet. In some embodiments the moisture vapor permeability of the backing layer increases when the backing layer becomes wet. The moisture vapor permeability of the wet backing layer may be up to about ten times more than the moisture vapor permeability of the dry backing layer.

The absorbent layer 221 may be of a greater area than the transmission layer 226, such that the absorbent layer overlaps the edges of the transmission layer 226, thereby ensuring that the transmission layer does not contact the backing layer 220. This provides an outer channel of the absorbent layer 221 that is in direct contact with the wound contact layer 222, which aids more rapid absorption of exudates to the absorbent layer. Furthermore, this outer channel ensures that no liquid is able to pool around the circumference of the wound cavity, which could seep through the seal around the perimeter of the dressing leading to the formation of leaks. As illustrated in Figures 2A-2B, the absorbent layer 221 may define a smaller perimeter than that of the backing layer 220, such that a boundary or border region is defined between the edge of the absorbent layer 221 and the edge of the backing layer 220.

As shown in Figure 2B, one embodiment of the wound dressing 100 comprises an aperture 228 in the absorbent layer 221 situated underneath the fluidic connector 110. In use, for example when oxygen or negative pressure is supplied to a wound covered by the dressing 100, a wound facing portion of the fluidic connector may thus come into contact with the transmission layer 226, which can thus aid in supplying oxygen or negative pressure to the wound site even when the absorbent layer 221 is filled with wound fluids. Some embodiments may have the backing layer 220 be at least partly adhered to the transmission layer 226. In some embodiments, the aperture 228 is at least 1-2 mm larger than the diameter of the wound facing portion of the fluidic connector 110, or the orifice 227.

In particular for embodiments with a single fluidic connector 110 and through hole, it may be preferable for the fluidic connector 110 and through hole to be located in an off-center position as illustrated in Figure 2A. Such a location may permit the dressing 100 to be positioned onto a patient such that the fluidic connector 110 is raised in relation to the remainder of the dressing 100. So positioned, the fluidic connector 110 and a filter 214 (described below) may be less likely to come into contact with wound fluids that could prematurely occlude the filter 214 so as to impair the transmission of oxygen or negative pressure to the wound site.

Turning now to the fluidic connector 110, preferred embodiments comprise a sealing surface 216, a bridge 211 (corresponding to bridge 120 in Figure 1) with a proximal end 130 and a distal end 140, and the filter 214. The sealing surface 216 preferably forms the applicator previously described that is sealed to the top surface of the wound dressing. In some embodiments, a bottom layer of the fluidic connector 110 may comprise the sealing surface 216. The fluidic connector 110 may further comprise an upper surface vertically spaced from the sealing surface 216, which in some embodiments is defined by a separate upper layer of the fluidic connector. In other embodiments, the upper surface and the lower surface may be formed from the same piece of material. In some embodiments, the sealing surface 216 may comprise at least one aperture 229 therein to communicate with the wound dressing. In some embodiments, the filter 214 may be positioned across the opening 229 in the sealing surface, and may span the entire opening 229. The sealing surface 216 may be configured for sealing the fluidic connector to the cover layer of the wound dressing, and may comprise an adhesive or weld. In some embodiments, the sealing surface 216 may be placed over an orifice in the cover layer. In other embodiments, the sealing surface 216 may be positioned over an orifice in the cover layer and an aperture in the absorbent layer 220, permitting the fluidic connector 110 to provide air flow through the transmission layer 226.

In some embodiments, the bridge 211 may comprise a first fluid passage 212 in communication with an oxygen source or source of negative pressure, the first fluid passage 212 comprising a porous material, such as a 3D knitted material, which may be the same or different than the porous layer 226 described previously. The bridge 211 is preferably encapsulated by at least one flexible film layer 208, 210 having a proximal and distal end and configured to surround the first fluid passage 212, the distal end of the flexible film being connected to the sealing surface 216. The filter 214 is configured to substantially prevent oxygen or wound exudate from entering the bridge. In some embodiments, a one-way valve can be used instead of or in addition to the filter to prevent wound fluid (or oxygen) from contaminating the oxygen source or negative pressure source.

Some embodiments may further comprise an optional second fluid passage positioned above the first fluid passage 212. For example, some embodiments may provide for an air leak may be disposed at the proximal end of the top layer 208 that is configured to provide an air path into the first fluid passage 212 and dressing 100 similar to the suction adapter as described in U.S. Patent No 8,801,685, filed December 30, 2011, entitled "APPARATUSES AND METHODS FOR NEGATIVE PRESSURE WOUND THERAPY".

Preferably, the fluid passage 212 is constructed from a compliant material that is flexible and that also permits fluid to pass through it if the spacer is kinked or folded over. Suitable materials for the fluid passage 212 include without limitation foams, including open-cell foams such as polyethylene or polyurethane foam, meshes, 3D knitted fabrics, non-woven materials, and fluid channels. In some embodiments, the fluid passage 212 may be constructed from materials similar to those described above in relation to the transmission layer 226. Advantageously, such materials used in the fluid passage 212 not only permit greater patient comfort, but may also provide greater kink resistance, such that the fluid passage 212 is still able to transfer oxygen to the wound or fluid from the wound toward the source of negative pressure while being kinked or bent.

In some embodiments, the fluid passage 212 may be comprised of a wicking fabric, for example a knitted or woven spacer fabric (such as a knitted polyester 3D fabric, Baltex 7970^{®}, or Gehring 879^{®}) or a nonwoven fabric. These materials selected are preferably suited to channeling wound exudate away from the wound and for supplying oxygen, negative pressure, vented air to the wound site, and may also confer a degree of kinking or occlusion resistance to the fluid passage 212. In some embodiments, the wicking fabric may have a three-dimensional structure, which in some cases may aid in wicking fluid or transmitting oxygen or negative pressure. In certain embodiments, including wicking fabrics, these materials remain open and capable of supplying oxygen or negative pressure to a wound area under the typical pressures used in oxygen or negative pressure therapy. In some embodiments, the wicking fabric may comprise several layers of material stacked or layered over each other, which may in some cases be useful in preventing the fluid passage 212 from expanding or collapsing under the application of positive or negative pressure. In other embodiments, the wicking fabric used in the fluid passage 212 may be between 1.5 mm and 6 mm; more preferably, the wicking fabric may be between 3 mm and 6 mm thick, and may be comprised of either one or several individual layers of wicking fabric. In other embodiments,
the fluid passage 212 may be between 1.2-3 mm thick, and preferably thicker than 1.5 mm. Some embodiments, for example a suction adapter used with a dressing which retains liquid such as wound exudate, may employ hydrophobic layers in the fluid passage 212, and only gases may travel through the fluid passage 212. Additionally, and as described previously, the materials used in the system are preferably conformable and soft, which may help to avoid pressure ulcers and other complications which may result from a wound treatment system being pressed against the skin of a patient.

Preferably, the filter 214 is impermeable to liquids, but permeable to gases, and is provided to act as a liquid barrier and to ensure that no liquids are able to escape from the wound dressing 100. The filter 214 may also function as a bacterial barrier. Typically the pore size is 0.2µm. Suitable materials for the filter material of the filter 214 include 0.2 micron Gore^{™} expanded PTFE from the MMT range, PALL Versapore^{™} 200R, and Donaldson^{™} TX6628. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter. The filter can be attached or sealed to the port or the cover film over the orifice. For example, the filter 214 may be molded into the fluidic connector 110, or may be adhered to one or both of the top of the cover layer and bottom of the suction adapter 110 using an adhesive such as, but not limited to, a UV cured adhesive.

It will be understood that other types of material could be used for the filter 214. More generally a microporous membrane can be used which is a thin, flat sheet of polymeric material, this contains billions of microscopic pores. Depending upon the membrane chosen these pores can range in size from 0.01 to more than 10 micrometers. Microporous membranes are available in both hydrophilic (water filtering) and hydrophobic (water repellent) forms. In some embodiments, filter 214 comprises a support layer and an acrylic co-polymer membrane formed on the support layer. Preferably the wound dressing 100 according to certain embodiments uses microporous hydrophobic membranes (MHMs). Numerous polymers may be employed to form MHMs. For example, the MHMs may be formed from one or more of PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing air or gas, including oxygen, to flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter 214 may also include an odor absorbent material, for example activated charcoal, carbon fiber cloth or Vitec Carbotec-RT Q2003073 foam, or the like. For example, an odor absorbent material may form a layer of the filter 214 or may be sandwiched between microporous hydrophobic membranes within the filter. The filter 214 thus enables gas to be exhausted through the orifice. Liquid, particulates and pathogens however are contained in the dressing. In some embodiments, the filter 214 is impermeable or substantially impermeable to oxygen so that oxygen supplied to the wound remains under the dressing 100.

Similar to the embodiments of wound dressings described above, some wound dressings comprise a perforated wound contact layer with silicone adhesive on the skin-contact face and acrylic adhesive on the reverse. Above this bordered layer sits a transmission layer or a 3D spacer fabric pad. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can include a high moisture vapor transmission rate (MVTR) film, pattern coated with acrylic adhesive. Adhesive can be provided across substantially entire backing layer, at the border of the wound dressing, or at another region or regions of the backing layer. The high MVTR film and wound contact layer encapsulate the transmission layer and absorbent layer, creating a perimeter border of approximately 20 mm. In some implementations, as disclosed herein, the backing layer can include film configured to switch moisture vapor permeability upon exposure to liquid. For example, the film can have relatively low moisture transmission rate, such as 600 (or less) to 1500 (or more) g/m²/24 hr, when dry. The film's moisture vapor transmission rate can increase to a relatively high level, such as 3000 (or less) to 15000 (or more) g/m²/24 hr, when contacted by liquid. The film can be IV3000 sold by Smith & Nephew. The backing layer can have a 10 mm aperture that overlies the aperture in the absorbent layer. Above the hole can be bonded a fluidic connector that comprises a liquid-impermeable, gas-permeable semi-permeable membrane (SPM) or filter that overlies the aforementioned apertures.

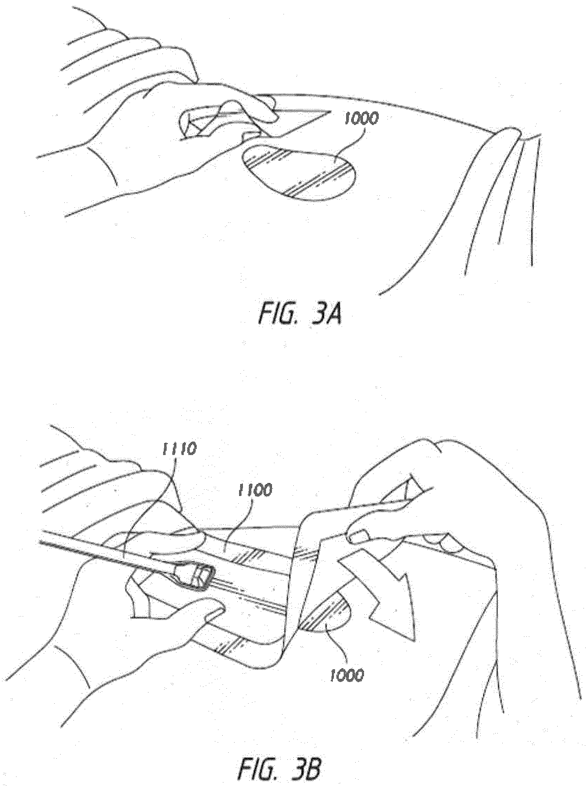

Figures 3A-D illustrate the use of an embodiment of a wound treatment system being used to treat a wound site on a patient. Figure 3A shows a wound site 1000 being cleaned and prepared for treatment. Here, the healthy skin surrounding the wound site 1000 is preferably cleaned and excess hair removed or shaved. The wound site 1000 may also be irrigated with sterile saline solution if necessary. Optionally, a skin protectant may be applied to the skin surrounding the wound site 1000. If necessary, a wound packing material, such as foam or gauze, may be placed in the wound site 1000. This may be preferable if the wound site 1000 is a deeper wound.

After the skin surrounding the wound site 1000 is dry, and with reference now to Figure 3B, the wound dressing 1100 may be positioned and placed over the wound site 1000. Preferably, the wound dressing 1100 is placed with the wound contact layer over or in contact with the wound site 1000. In some embodiments, an adhesive layer is provided on the lower surface of the wound contact layer, which may in some cases be protected by an optional release layer to be removed prior to placement of the wound dressing 1100 over the wound site 1000. Preferably, the dressing 1100 is positioned such that the fluidic connector 1110 is in a raised position with respect to the remainder of the dressing 1100 so as to avoid fluid pooling around the port. In some embodiments, the dressing 1100 is positioned so that the fluidic connector 1110 is not directly overlying the wound, and is level with or at a higher point than the wound. To help ensure adequate sealing for TNP, the edges of the dressing 1100 are preferably smoothed over to avoid creases or folds.

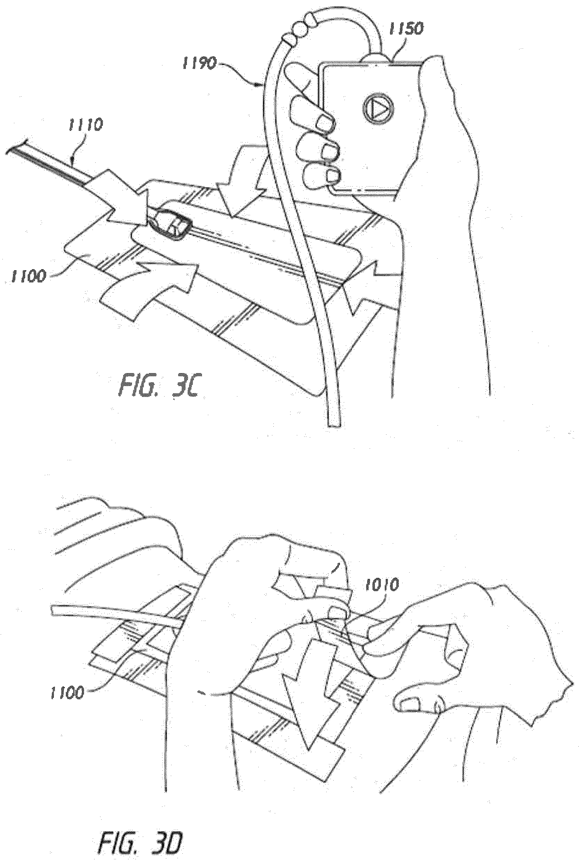

With reference now to Figure 3C, the dressing 1100 is connected to an oxygen reservoir or source 1150. The source 1150 is configured to supply oxygen to the wound site via the dressing 1100, and typically through a conduit. In some embodiments, and as described herein, a fluidic connector 1110 may be used to join the conduit 1190 from the source 1150 to the dressing 1100. Where the fluidic connector is adhered to the top layer of the wound dressing, a length of tubing may be coupled at a first end of the fluidic connector such that the tubing, or conduit, extends away from the fluidic connector parallel to the top of the dressing. In some embodiments, the conduit may comprise a fluidic connector. It is expressly contemplated that a conduit may be a soft bridge, a hard tube, or any other apparatus which may serve to transport fluid.

Turning to Figure 3D, additional fixation strips 1010 may also be attached around the edges of the dressing 1100. Such fixation strips 1010 may be advantageous in some situations so as to provide additional sealing against the skin of the patient surrounding the wound site 1000. For example, the fixation strips 1010 may provide additional sealing for when a patient is more mobile. In some cases, the fixation strips 1010 may be used prior to activation of the source 1150, particularly if the dressing 1100 is placed over a difficult to reach or contoured area.

Treatment of the wound site 1000 preferably continues until the wound has reached a desired level of healing. In some embodiments, it may be desirable to replace the dressing 1100 after a certain time period has elapsed, or if the dressing is full of wound fluids. During such changes, the source 1150 may be kept, with just the dressing 1100 being changed.

In some implementations, wound dressing embodiments described herein are well-suited for sustained delivery of oxygen over the entire or substantially entire surface of the wound. One or more of the transmission layer (or the ADL), through hole, and perforated wound contact layer can facilitate delivery of oxygen from the oxygen source over the entire or substantially entire surface of the wound. In addition, one or more of the transmission layer (or the ADL), the absorbent layer, and moisture vapor permeable backing layer can facilitate efficient removal of fluid from the wound so that oxygen can be more effectively delivered to the wound. Accordingly, wound dressing embodiments described herein can help keep the wound moist to facilitate dissolution of oxygen, while substantially preventing fluid pooling which adversely impacts dissolution of oxygen. The filter (or one-way valve as described herein) can facilitate protecting the oxygen source from wound fluid. Further, the pressure sensitive adhesive of the wound contact layer can provide a lasting, gas-tight seal over the wound. The pressure sensitive adhesive can be silicone acrylic, acrylic adhesive, or the like. In addition, adhesive on the backing layer, fixation strips, or the like can provide an additional or alternative gas-tight seal.

In certain embodiments, a gasket can additionally or alternatively be used to achieve a gas-tight seal. The gasket can be positioned on the backing layer. The gasket can be formed from hydrocolloids, hydrogels, or the like.

### Oxygen Therapy in Combination with Negative Pressure

In certain implementations, negative pressure wound therapy can be additionally or alternatively used with oxygen therapy. In some embodiments, negative pressure can be delivered sequentially or simultaneously with oxygen therapy. A negative pressure source can be fludically connected to the wound dressing via another orifice or port in the dressing as described herein. A fluidic connector, such as any of the connector described herein, can be used to connect the pump the dressing. Alternatively, a Y-connector can be utilized to connect both the pump and the oxygen source to the wound dressing via a single orifice. Additional embodiments of fluidic connections are described in U.S. Patent No. 9,061,095, titled "Wound dressing and method of use," issued on June 23, 2015.

A negative pressure source can include a pump. The pump can be actuated by one or more of a piezoelectric transducer, voice coil actuator, motor, hand or foot operated mechanism, or the like.

The pump may comprise a canister or other container for the storage of wound exudates and other fluids that may be removed from the wound. A canister or container may also be provided separate from the pump. In some embodiments, the pump can be a canisterless pump such as the PICO^{™} pump, as sold by Smith & Nephew.

In some embodiments, sequential oxygen and negative pressure therapy cycles between periods of oxygen delivery and periods of applying negative pressure. Respective therapies can be applied over any suitable time duration to effectively facilitate wound healing.

In certain implementations, simultaneous oxygen and negative pressure therapy can be utilized. This may be accomplished with the use of multiple ports for oxygen delivery and negative pressure application respectively. In some cases, oxygen can be delivered at 100% of target pressure (such as, 20 mmHg), while negative pressure can be delivered at less than 100% of target pressure, such as, at 80% of target pressure (or less or more than 80%). Target negative pressure can be selected or preset. For example, target negative pressure can be -80 mmHg. In certain situations, both therapies can be delivered at 100% of target pressure levels. In some embodiments, oxygen therapy can be delivered at less than 100% of target pressure, while negative pressure can be delivered at 100% of target pressure.

In some embodiments, oxygen can be delivered at a selected flow rate while applying a target negative pressure, such as -80 mmHg or another suitable level. Flow rate of oxygen can be selected to provide a suitable level of positive pressure into the fluid flow path to facilitate movement of fluid through the fluid flow path, which can prevent or reduce stagnation of the flow. In other words, flow rate of oxygen can be selected to provide a suitable controlled gas leak. Flow rate of oxygen can be selected to achieve a balance between the oxygen therapy, which supplies positive pressure, and negative pressure therapy. Flow rate of oxygen can be selected based on one or more of volume of the wound dressing, volume of the tubing or lumen(s) in the fluid flow path, rate of flow provided by the negative pressure source (which may depend on the target pressure), volume of a canister (if present), or the like. In such embodiments, oxygen can be delivered at or close to 100% of target pressure, which can help facilitate better absorption by the wound.

### Therapy Control

In some embodiments, the system can utilize one or more sensors positioned in the fluid flow path to facilitate control or oxygen or negative pressure delivery. For example, a pressure sensor can be positioned in the fluid flow path, such as in the inlet of the oxygen source or negative pressure source. Feedback from the pressure sensor can be used to regulate delivery of oxygen, negative pressure, or detect and signal presence of various operating conditions, such as leaks, blockages, or the like in the fluid flow path. One or more of the oxygen source of negative pressure source can include control circuitry, such as one or more controllers, to monitor conditions in the fluid flow path or regulate delivery of oxygen or negative pressure therapy (for example, regulate sequential or simultaneous therapy). In some implementations, one or more oxygen sensors can be positioned in the fluid flow path, such as in the wound, to determine oxygen level(s) or regulate oxygen delivery. One or more flow sensor or meters can be used.

### Other Variations

In some embodiments, a source of oxygen or negative pressure (such as a pump) and some or all other components of the wound treatment system, such as power source(s), sensor(s), connector(s), user interface component(s) (such as button(s), switch(es), speaker(s), screen(s), etc.) and the like, can be integral with the wound dressing. In some embodiments, the components can be integrated below, within, on top of, or adjacent to the backing layer. In some embodiments, the wound dressing can include a second cover layer or a second filter layer for positioning over the layers of the wound dressing and any of the integrated components. The second cover layer can be the upper most layer of the dressing or can be a separate envelope that enclosed the integrated components of the wound treatment system.

In some instances, a wound dressing can include a wound filler placed in a wound cavity. The wound filler can be one or more of foam (such as, reticulated foam), gauze, antimicrobial material (such as, ACTICOAT sold by Smith & Nephew), gelling fiber (such as, DURAFIBER sold by Smith & Nephew), or the like. The wound filler can be sealed by a suitable backing layer to provide a substantially gas impermeable seal. In certain embodiments, a wound dressing can include a non-adherent wound contact layer, such as CUTICERIN sold by Smith & Nephew, MEPITEL sold by Mölnlycke Health Care, or the like.

In certain implementations, other gases or fluids can be supplied to the wound in addition to or instead of oxygen. For example, ozone can be supplied in gaseous or liquid form. As another example, nitric oxide can be supplied to the wound in control amounts to help with healing.

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes may be described in terms of determining whether a value meets or does not meet a particular threshold, the blocks can be similarly understood, for example, in terms of a value (i) being below or above a threshold or (ii) satisfying or not satisfying a threshold.

All of the features disclosed in this specification (including any accompanying exhibits, claims, abstract and drawings), or all of the steps of any method or process so disclosed, may be combined in any combination, except combinations where at least some of such features or steps are mutually exclusive.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those described or illustrated. Depending on the embodiment, certain of the steps described above may be removed, others may be added. For example, the actual steps or order of steps taken in the disclosed processes may differ from those described or illustrated. Depending on the embodiment, certain of the steps described above may be removed, others may be added. For instance, the various components may be implemented as software or firmware on a processor, controller, ASIC, FPGA, or dedicated hardware. Hardware components, such as processors, ASICs, FPGAs, and the like, can include logic circuitry. Furthermore, the features and attributes of the specific embodiments disclosed above may be combined in different ways to form additional embodiments, all of which fall within the scope of the present disclosure.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

## Claims

1. A wound treatment apparatus comprising:
a wound dressing configured to be positioned over a wound to provide a substantially fluid impermeable seal over the wound, the wound dressing comprising:
a wound contact layer configured to be positioned in contact with the wound;
**characterised in that**:
the wound dressing further comprises:
a transmission layer positioned above the wound contact layer, the transmission layer configured to transmit wound fluid away from the wound;
an absorbent layer positioned above the transmission layer, the absorbent layer configured to absorb wound fluid; and
a backing layer positioned above the absorbent layer, the backing layer comprising an orifice; and
the wound treatment apparatus further comprises an oxygen source configured to supply oxygen to the wound through the orifice.

2. The wound treatment apparatus of any of the preceding claims, wherein the transmission layer comprises at least one of a material with three dimensional structure or an acquisition distribution layer.

3. The wound treatment apparatus of any of the preceding claims, wherein the absorbent layer comprises an orifice configured to provide direct fluidic communication between the oxygen source and the transmission layer.

4. The wound treatment apparatus of any of the preceding claims, wherein the wound contact layer comprises adhesive on a wound facing side, the adhesive configured to provide a substantially gas tight seal over the wound.

5. The wound treatment apparatus of claim 4, wherein the adhesive comprises silicone adhesive.

6. The wound treatment apparatus of any of the preceding claims, wherein the wound contact layer comprises a plurality of slits configured to distribute oxygen over the wound and further configured to transmit wound fluid away from the wound.

7. The wound treatment apparatus of any of the preceding claims, wherein the backing layer is substantially oxygen and fluid impermeable.

8. The wound treatment apparatus of claim 7, wherein the backing layer comprises ethylene vinyl alcohol (EVA).

9. The wound treatment apparatus of any of the preceding claims, wherein the backing layer comprises another orifice configured to facilitate supply of negative pressure to the wound.

10. The wound treatment apparatus of claim 9, further comprising a negative pressure source configured to supply negative pressure to the wound through the another orifice.

11. The wound treatment apparatus of any of the preceding claims, wherein the wound dressing further comprises a filter configured to prevent wound fluid from reaching the oxygen source.

12. The wound treatment apparatus of any of the preceding claims, further comprising a pressure sensor positioned in a fluid flow path comprising the oxygen source and the wound dressing and a controller configured to regulate supply of oxygen based on feedback from the pressure sensor.

13. The wound treatment apparatus of claim 12, wherein the controller is further configured to regulate application of negative pressure to the wound.

14. The wound treatment apparatus of any of claims 12 or 13, wherein the controller is further configured to regulate application of simultaneous supply of oxygen and negative pressure to the wound.

15. The wound treatment apparatus of claims 12 or 13, wherein the controller is further configured to regulate application of sequential supply of oxygen and negative pressure to the wound.

## Patentansprüche

1. Eine Wundbehandlungsvorrichtung, die Folgendes beinhaltet:
einen Wundverband, der konfiguriert ist, um über einer Wunde positioniert zu werden, um eine im Wesentlichen fluidundurchlässige Abdichtung über der Wunde bereitzustellen, wobei der Wundverband Folgendes beinhaltet:
eine Wundkontaktschicht, die konfiguriert ist, um in Kontakt mit der Wunde positioniert zu werden;
**dadurch gekennzeichnet, dass**:
der Wundverband ferner Folgendes beinhaltet:
eine Durchlassschicht, die oberhalb der Wundkontaktschicht positioniert ist, wobei die Durchlassschicht konfiguriert ist, um Wundfluid von der Wunde weg durchzulassen;
eine absorbierende Schicht, die oberhalb der Durchlassschicht positioniert ist, wobei die absorbierende Schicht konfiguriert ist, um Wundfluid zu absorbieren; und
eine Unterstützungsschicht, die oberhalb der absorbierenden Schicht positioniert ist, wobei die Unterstützungsschicht eine Öffnung beinhaltet; und
die Wundbehandlungsvorrichtung ferner eine Sauerstoffquelle beinhaltet, die konfiguriert ist, um die Wunde durch die Öffnung mit Sauerstoff zu versorgen.

2. Wundbehandlungsvorrichtung gemäß einem der vorhergehenden Ansprüche, wobei die Durchlassschicht mindestens eines von einem Material mit einer dreidimensionalen Struktur oder einer Aufnahmeverteilungsschicht beinhaltet.

3. Wundbehandlungsvorrichtung gemäß einem der vorhergehenden Ansprüche, wobei die absorbierende Schicht eine Öffnung beinhaltet, die konfiguriert ist, um direkte Fluidkommunikation zwischen der Sauerstoffquelle und der Durchlassschicht bereitzustellen.

4. Wundbehandlungsvorrichtung gemäß einem der vorhergehenden Ansprüche, wobei die Wundkontaktschicht einen Klebstoff auf einer der Wunde zugewandten Seite beinhaltet, wobei der Klebstoff konfiguriert ist, um eine im Wesentlichen gasdichte Abdichtung über der Wunde bereitzustellen.

5. Wundbehandlungsvorrichtung gemäß Anspruch 4, wobei der Klebstoff Silikonklebstoff beinhaltet.

6. Wundbehandlungsvorrichtung gemäß einem der vorhergehenden Ansprüche, wobei die Wundkontaktschicht eine Vielzahl von Schlitzen beinhaltet, die konfiguriert ist, um Sauerstoff über der Wunde zu verteilen, und ferner konfiguriert ist, um Wundfluid von der Wunde weg durchzulassen.

7. Wundbehandlungsvorrichtung gemäß einem der vorhergehenden Ansprüche, wobei die Unterstützungsschicht im Wesentlichen sauerstoff- und fluidundurchlässig ist.

8. Wundbehandlungsvorrichtung gemäß Anspruch 7, wobei die Unterstützungsschicht Ethylenvinylalkohol (EVA) beinhaltet.

9. Wundbehandlungsvorrichtung gemäß einem der vorhergehenden Ansprüche, wobei die Unterstützungsschicht eine weitere Öffnung beinhaltet, die konfiguriert ist, um die Versorgung der Wunde mit Unterdruck zu erleichtern.

10. Wundbehandlungsvorrichtung gemäß Anspruch 9, die ferner eine Unterdruckquelle beinhaltet, die konfiguriert ist, um die Wunde durch die weitere Öffnung mit Unterdruck zu versorgen.

11. Wundbehandlungsvorrichtung gemäß einem der vorhergehenden Ansprüche, wobei der Wundverband ferner ein Filter beinhaltet, das konfiguriert ist, um zu verhindern, dass Wundfluid die Sauerstoffquelle erreicht.

12. Wundbehandlungsvorrichtung gemäß einem der vorhergehenden Ansprüche, das ferner einen Drucksensor, der in einem Fluidströmungsweg positioniert ist, der die Sauerstoffquelle und den Wundverband beinhaltet, und eine Steuereinheit, die konfiguriert ist, um die Versorgung mit Sauerstoff basierend auf einer Rückmeldung von dem Drucksensor zu regeln, beinhaltet.

13. Wundbehandlungsvorrichtung gemäß Anspruch 12, wobei die Steuereinheit ferner konfiguriert ist, um die Anwendung von Unterdruck auf die Wunde zu regeln.

14. Wundbehandlungsvorrichtung gemäß einem der Ansprüche 12 oder 13, wobei die Steuereinheit ferner konfiguriert ist, um die Anwendung einer gleichzeitigen Versorgung der Wunde mit Sauerstoff und Unterdruck zu regeln.

15. Wundbehandlungsvorrichtung gemäß den Ansprüchen 12 oder 13, wobei die Steuereinheit ferner konfiguriert ist, um die Anwendung einer sequentiellen Versorgung der Wunde mit Sauerstoff und Unterdruck zu regeln.

## Revendications

1. Un appareil de traitement de plaie comprenant :
un pansement pour plaie configuré pour être positionné par-dessus une plaie afin de fournir un joint d'étanchéité substantiellement imperméable aux fluides par-dessus la plaie, le pansement pour plaie comprenant :
une couche de contact avec la plaie, configurée pour être positionnée en contact avec la plaie ;
**caractérisé en ce que** :
le pansement pour plaie comprend en outre :
une couche de transmission positionnée au-dessus de la couche de contact avec la plaie, la couche de transmission étant configurée pour transmettre du fluide de plaie à l'écart de la plaie ;
une couche absorbante positionnée au-dessus de la couche de transmission, la couche absorbante étant configurée pour absorber du fluide de plaie ; et
une couche dorsale positionnée au-dessus de la couche absorbante, la couche dorsale comprenant un orifice ; et
l'appareil de traitement de plaie comprend en outre une source d'oxygène configurée pour apporter de l'oxygène à la plaie à travers l'orifice.

2. L'appareil de traitement de plaie de n'importe lesquelles des revendications précédentes, dans lequel la couche de transmission comprend au moins soit un matériau avec une structure en trois dimensions, soit une couche d'acquisition-distribution.

3. L'appareil de traitement de plaie de n'importe lesquelles des revendications précédentes, dans lequel la couche absorbante comprend un orifice configuré pour fournir une communication fluidique directe entre la source d'oxygène et la couche de transmission.

4. L'appareil de traitement de plaie de n'importe lesquelles des revendications précédentes, dans lequel la couche de contact avec la plaie comprend un adhésif sur un côté tourné vers la plaie, l'adhésif étant configuré pour fournir un joint d'étanchéité substantiellement étanche aux gaz par-dessus la plaie.

5. L'appareil de traitement de plaie de la revendication 4, dans lequel l'adhésif comprend un adhésif en silicone.

6. L'appareil de traitement de plaie de n'importe lesquelles des revendications précédentes, dans lequel la couche de contact avec la plaie comprend une pluralité de fentes configurées pour distribuer l'oxygène par-dessus la plaie et configurées en outre pour transmettre du fluide de plaie à l'écart de la plaie.

7. L'appareil de traitement de plaie de n'importe lesquelles des revendications précédentes, dans lequel la couche dorsale est substantiellement imperméable à l'oxygène et aux fluides.

8. L'appareil de traitement de plaie de la revendication 7, dans lequel la couche dorsale comprend de l'éthylène alcool de vinyle (EVA).

9. L'appareil de traitement de plaie de n'importe lesquelles des revendications précédentes, dans lequel la couche dorsale comprend un autre orifice configuré pour faciliter l'apport de pression négative à la plaie.

10. L'appareil de traitement de plaie de la revendication 9, comprenant en outre une source de pression négative configurée pour apporter une pression négative à la plaie à travers l'autre orifice.

11. L'appareil de traitement de plaie de n'importe lesquelles des revendications précédentes, dans lequel le pansement pour plaie comprend en outre un filtre configuré pour empêcher du fluide de plaie d'atteindre la source d'oxygène.

12. L'appareil de traitement de plaie de n'importe lesquelles des revendications précédentes, comprenant en outre un capteur de pression positionné dans une voie d'écoulement de fluide comprenant la source d'oxygène et le pansement pour plaie et un contrôleur configuré pour réguler l'apport d'oxygène sur la base d'un retour du capteur de pression.

13. L'appareil de traitement de plaie de la revendication 12, dans lequel le contrôleur est en outre configuré pour réguler l'application de pression négative sur la plaie.

14. L'appareil de traitement de plaie de n'importe lesquelles des revendications 12 ou 13, dans lequel le contrôleur est en outre configuré pour réguler l'application d'un apport simultané d'oxygène et de pression négative sur la plaie.

15. L'appareil de traitement de plaie des revendications 12 ou 13, dans lequel le contrôleur est en outre configuré pour réguler l'application d'un apport séquentiel d'oxygène et de pression négative sur la plaie.
